# EUROPEAN PATENT APPLICATION

(11) **EP 1 683 805 A1**
(43) Date of publication of application: **26.07.2006**
(21) Application number: 04792603.5
(22) Date of filing: 19.10.2004
(51) Int. Cl.: C07H 17/065, A61K 31/7048, A61K 36/18, A23L 1/30, C12N 9/99, A61P 3/10, A61P 9/10, A61P 13/00, A61P 25/00, A61P 27/02, A61P 43/00

(54) **NOVEL INHIBITOR FOR ADVANCED GLYCATION ENDPRODUCT FORMATION AND ALDOSE REDUCTASE INHIBITOR**

(30) Priority: 24.10.2003 JP 2003365190
(71) Applicant: Meiji Seika Kaisha, Ltd., Tokyo 104-8002 (JP)
(72) Inventor: YOSHIKAWA, Masayuki, Kyoto Pharmaceutical Uni., Kyoto-shi, Kyoto 607-8412 (JP); MATSUDA, Hisashi, Kyoto Pharmaceutical Uni., Kyoto-shi, Kyoto 607-8412 (JP); YAMAGISHI, Megumi, Meiji Seika Kaisha, Ltd., Sakado-shi, Saitama 350-0289 (JP); MATSUMOTO, Hitoshi, Meiji Seika Kaisha, Ltd., Sakado-shi, Saitama 350-0289 (JP)
(74) Representative: Gillard, Richard Edward
(86) International application number: PCT/JP2004/015434
(87) International publication number: WO 2005/040182

(57) **Abstract**

Provided are: an inhibitor of the formation of an advanced glycation end product (AGE) and aldose reductase for treating and preventing both various diseases relating to AGE formation, such as complications related to diabetes and various diseases relating to aldose reductase inhibition; pharmaceutical preparations, compositions, and food and drink that contain these active ingredients; and pharmaceutical preparations and foods prepared by blending compositions containing anthocyanin, because such anthocyanin obtained through condensation or extraction from raw plant materials or the like is useful as an inhibitor of AGE formation and aldose reductase.

## Description

### Technical Field

The present invention relates to a novel inhibitor of the formation of an advanced glycation end product (hereinafter, may also be abbreviated as AGE), a novel inhibitor of aldose reductase, compositions containing these inhibitors, pharmaceutical preparations containing such compositions, and foods containing such compositions. Specifically, the present invention relates to compositions that contain these novel compounds as active ingredients and are useful as preventive and therapeutic agents against diabetic complications (e.g., diabetic nephropathy, diabetic neuropathy, diabetic retinopathy, cataract, and macroangiopathy) or against various other diseases relating to AGE formation (e.g., Alzheimer's disease, amyotrophic lateral sclerosis, dialysis amyloidosis, and arthrorheumatism), pharmaceutical preparations containing such compositions, and foods containing such compositions.

### Background Art

The number of diabetes patients has continuously grown in recent years. According to the statistics as reported by the Ministry of Health, Labour and Welfare in 1998, the affected population was estimated to be 690 million in Japan, and 1,400 million if the number of people who might be affected with diabetes was included. A factor that directly influences on the life expectancy and the quality of life (QOL) of diabetes patients is not primary deficiency in insulin action, but is angiopathy, and specifically, vascular complication, which is secondarily generated at each part of the whole body as a result of hyperglycemia. Therefore, elucidation of ways of overcoming complications related to diabetes is a national research object that requires urgent solution.

In the case of diabetes involving a blood glucose level that is significantly higher than that in a normal case, reactions of glucose with some proteins (e.g., hemoglobin, lens crystalline, and collagen) cause the formation of advanced glycation end products (AGE). The formation of AGE then causes complications relating to diabetes (e.g., nephropathy, microangiopathy, endothelial dysfunction, and other organ dysfunctions). Furthermore, it also weakens the activities of some growth factors (e.g., basic fibroblast growth factor). AGE is an advanced product that is obtained through a reaction of an amino group of a protein with an aldehyde group of a reducing sugar to give a Schiff base and an Amadori rearrangement product (early stage product) and then through reactions such as dehydration, oxidation, and condensation. Unlike intra-tissue normal proteins, AGE is supplemented through metabolic turnover at slower rate than such proteins. The mechanisms of diabetes cases where evidence of microangiopathy and macroangiopathy exists have not been elucidated. It has been demonstrated that in such diabetes cases, evidence of oxidative stress is also exhibited. Furthermore, it has also been suggested that AGE is involved in various complications accompanying diabetes or aging. AGE is also known to bind to cell surface receptors such as receptors expressed on the surfaces of cells such as monocytes, macrophages, neurons, smooth muscle cells, and endothelial cells. AGE is thought to interact with such receptors and has various physiological and biological actions on living bodies and cells.

Methods for treating or preventing complications related to diabetes and the like by inhibiting AGE formation or degrading the formed AGE have been expected. Some methods for such purposes have been disclosed.

A typical example of an AGE formation inhibitor (Maillard reaction inhibitor) is aminoguanidine disclosed in JP Patent Publication (Kokoku) No. 6-67827 B (1994) (Patent document 1). Furthermore, regarding a pharmaceutical preparation, JP Patent Publication (Kokai) No. 2002-302472 A (Patent document 2) reports a novel AGE formation inhibitor (Millard reaction inhibitor). JP Patent Publication (Kohyo) No. 2002-543118 A (Patent document 3) reports Pentoxifyllin, Pioglitazone, and Metformin as AGE formation inhibitors. According to JP Patent Publication (Kohyo) No. 2002-541139 A (Patent document 4), it has been discovered that aryl and heterocyclic ureide or aryl and a derivative of a heterocyclic carboxamide phenoxyisobutyric acid inhibit non-enzymatic glycation of protein, which often results in AGE formation and crosslinking formation. However, these compounds have concern over side effects, so that long-term ingestion of such compounds in large quantities is restricted. Thus, a compound that enables safe and long-term ingestion and enables prevention or treatment of diabetic complications has been desired.

Regarding foods, as represented by an α-glucosidase inhibitor prepared using a product obtained by heating wheat bran according to JP Patent Publication (Kokai) No. 2000-186044 A (Patent document 5), methods for treating and preventing diabetes based on an effect of inhibiting α-glucosidase have been reported. However, AGE formation-inhibiting action has never been reported. Furthermore, there has been no knowledge concerning methods for preventing or treating complications related to diabetes.

Moreover, aldose reductase is capable of converting, in a warm blooded animal such as a human, aldose such as glucose and galactose through contact into equivalent forms of alditol such as sorbitol and galactitol, respectively. Alditol penetrates a cell membrane with difficulty. Once alditol is formed, it tends to be removed only by the subsequent process of metabolism. As a result, alditol tends to be accumulated inside the cells wherein it is formed. This causes an elevation in internal osmotic pressure and that can be enough to destroy or damage the functions of cells. Furthermore, an increased alditol level results in abnormal levels of their intermediate metabolites that can impair or damage the functions of cells. Aldose reductase has relatively low affinity for aldose and can generally exert its effect only in the presence of aldose at a relatively high concentration. Such high concentrations of aldose are found in clinical states of diabetes (excess glucose) and galactosemia (excess galactose). Therefore, an aldose reductase inhibitor is useful for alleviating or preventing the exertion of peripheral effects of diabetes or galactosemia (which can be partially based on sorbitol or galactitol accumulation in tissues such as those of the eyes, nerves, and kidneys). Examples of such peripheral effects include macular oedama, cataract, retinopathy, neuropathy, and impaired neural conduction. Numerous aldose reductase inhibitors have been discovered and clinically evaluated. However, demand for another inhibitor continues to exist.

The primary ingredients of compounds or compositions and/or food and drink used in the present invention are anthocyanins. Anthocyanins are known to have action to remove active oxygen. As per a patent for which the present inventors have separately applied (WO01/01798 (Patent document 6)), anthocyanins are known to have an effect of improving visual function, an effect of improving the blood fluidity, and the like. Furthermore, as disclosed in JP Patent Publication (Kokai) No. 2000-32954 A (Patent document 7), an effect of preventing cataracts based on an anti-oxidation function is expected. However, such publication discloses nothing about AGE-inhibiting activity, aldose-reductase-inhibiting activity, or diabetic complication. JP Patent Publication (Kokai) No. 10-59846 A (Patent document 8) discloses the cataract-preventing effect of a grape-derived proanthocyanidin oligomer. However it discloses nothing about AGE-inhibiting activity, aldose-reductase-inhibiting activity, or diabetic complications. JP Patent Publication No. 2967523 (Patent document 9) reports an ophthalmic pharmaceutical composition comprising anthocyanidin as an active ingredient. However, it discloses nothing about AGE-inhibiting activity or aldose-reductase-inhibiting activity. Furthermore, anthocyanidin is not glycoside, and thus there is a difference from the present invention.

Accordingly, the present inventors have discovered that anthocyanins have both AGE-inhibiting activity and aldose-reductase-inhibiting activity. Thus, the present inventors could have discovered the effect of anthocyanins in prevention and treatment of diabetic complications and the like.
Patent document 1: JP Patent Publication (Kokoku) No. 6-67827 (1994) B
Patent document 2: JP Patent Publication (Kokai) No. 2002-302472 A
Patent document 3: JP Patent Publication (Kohyo) No. 2002-543118 A
Patent document 4: JP Patent Publication (Kohyo) No. 2002-541139 A
Patent document 5: JP Patent Publication (Kokai) No. 2000-186044 A
Patent document 6: WO01/01798
Patent document 7: JP Patent Publication (Kokai) No. 2000-32954 A
Patent document 8: JP Patent Publication (Kokai) No. 10-59846 A
Patent document 9: JP Patent Publication No. 2967523

### Disclosure of the Invention

Objects to be achieved by the present invention

Under previously described circumstances, the present inventors have discovered compounds that structurally differ from the AGE formation inhibitors that have been reported thus far and that have both strong AGE-formation-inhibiting activity and aldose-reductase-inhibiting activity. An object of the present invention is to provide compositions comprising such compounds, which are useful as preventive and therapeutic agents against the peripheral effects of diabetes or galactosemia (e.g., macular oedama, cataract, retinopathy, neuropathy, and impaired neural conduction) based on preventive and therapeutic action against diabetic complications relating to AGE formation (e.g., diabetic nephropathy, diabetic neuropathy, diabetic retinopathy, cataract, and macroangiopathy) or against other various diseases relating to AGE formation (e.g., Alzheimer's disease and amyotrophic lateral sclerosis, dialysis amyloidosis, and arthrorheumatism) and aldose-reductase-inhibiting action. Still another object of the present invention is to provide pharmaceutical preparations or food and drink comprising such compositions.

### Means to achieve the objects

As a result of intensive studies to obtain compounds that are derived from natural substances, that can be obtained in large amounts, and that can inhibit AGE formation, the present inventors have discovered compounds that strongly inhibit AGE formation. The present inventors have also discovered that the compounds also have aldose-reductase-inhibiting activity. Thus, the present inventors have completed the present invention.

A compound that can be used as the inhibitor of the present invention is anthocyanin, which is a form of glycoside. The generic name of a compound comprising a backbone as shown in the following structural formula is anthocyan. Anthocyan comprising only aglycon is particularly referred to as anthocyanidin; and anthocyan having sugar bound thereto as glycoside is particularly referred to as anthocyanin. Examples of anthocyanidin include the following delphinidin, cyanidin, malvidin, pelargonidin, peonidin, and petunidin depending on their side chains. For example, anthocyanin having glucose bound thereto as glycoside can also be referred to as anthocyanidin glucoside. Furthermore, the generic name of anthocyanidin and anthocyanin is anthocyan. 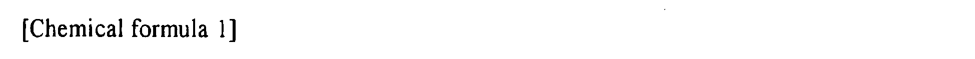 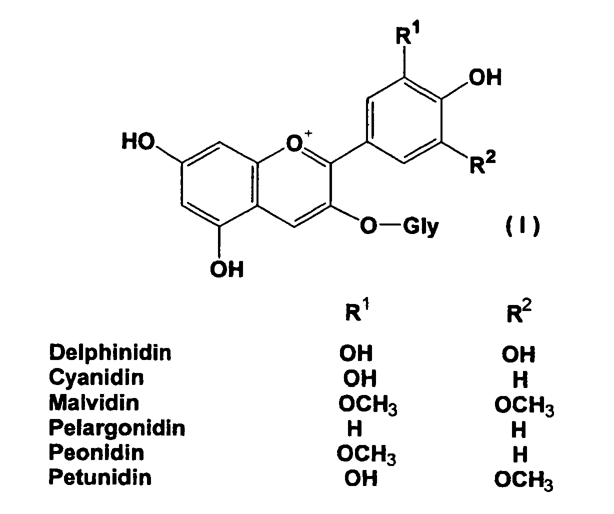 [wherein, R¹ and R² are the same or different and are each a hydrogen atom, a hydroxyl group, or a methoxy group and Gly denotes a saccharide group such as glucose, rutinose, arabinose, galactose, or sopholose.]

Anthocyans are broadly present in nature. Anthocyans are used in foods mainly as natural dyes or, because of their functionality, are also broadly used in pharmaceutical preparations, quasi-drugs, cosmetics, and the like in Europe. The use of anthocyan as a cicatrizant is as disclosed in JP Patent Publication (Kokoku) No. 59-53883 B (1984), for example. Alternatively, it has been discovered that anthocyan has valuable pharmacological properties for the treatment of peripheral vessel disease using blue-berry-derived anthocyanin as disclosed in JP Patent Publication (Kokai) No. 3-81220 A (1991). Recently also in Japan, the use of anthocyanin based on its functionality is attracting attention in addition to the use of the same as a dye. The present inventors have also discovered some useful efficacies of anthocyanin derived from cassis (English name: Black currant; Japanese name: *Kurofusasuguri*) and have applied for a related patent (PCT/JP00/04337).

In particular, the present inventors have discovered that 4 types of compounds including 3-glucoside and rutinoside of delphinidin and cyanidin can strongly inhibit AGE formation with a concentration as low as approximately one-tenth of that of aminoguanidine, which is a control agent for comparison. Therefore, through the ingestion of the effective dose of such anthocyanin ingredients, diabetic complications (e.g., diabetic nephropathy, diabetic neuropathy, diabetic retinopathy, cataract, and macroangiopathy) or other various diseases relating to AGE formation (e.g., Alzheimer's disease, amyotrophic lateral sclerosis, dialysis amyloidosis, and arthrorheumatism) can be prevented or treated.

Furthermore, such anthocyanin ingredients also have aldose-reductase-inhibiting activity. Thus, the peripheral effects of diabetes or galactosemia (e.g., macular oedama, cataract, retinopathy, neuropathy, and impaired neural conduction) can be prevented or treated using such anthocyanin ingredients.

The present invention is as follows:
[1] an inhibitor of the formation of advanced glycation end products, which comprises anthocyanin;
[2] the inhibitor of the formation of advanced glycation end products according to [1], wherein the anthocyanin is glycoside of delphinidin or cyanidin;
[3] an inhibitor of aldose reductase, which comprises anthocyanin;
[4] the inhibitor of aldose reductase according to [3], wherein the anthocyanin is glysocide of delphinidin or cyanidin;
[5] a composition, which comprises anthocyanin and has activity of inhibiting advanced glycation end product formation and aldose-reductase-inhibiting activity;
[6] a composition, which contains the inhibitor of the formation of advanced glycation end products according to [1] or [2] or the inhibitor of aldose reductase according to [3] or [4];
[7] the composition according to [6], which is a medicament containing anthocyanin prepared from berries;
[8] the composition according to [6], which is a medicament containing anthocyanin prepared from cassis;
[9] the composition according to [6], which is a food containing anthocyanin prepared from berries;
[10] the composition according to [6], which is a food containing anthocyanin prepared from cassis;
[11] a preventive or therapeutic food against disease caused by the formation of advanced glycation end products or aldose reductase activity, which comprises anthocyanin; and
[12] the preventive or therapeutic food according to [11], wherein the above disease is diabetes or a related complication tereof.

This description includes part or all of the contents as disclosed in the description or drawings of Japanese Patent Application No. 2003-365190, which is a priority document of the present application.

### Effect of the Invention

As shown in the examples of the present invention, the anthocyanin of the present invention has AGE-formation-inhibiting activity that is better than that of aminoguanidine. Furthermore, the anthocyanin of the present invention also has aldose-reductase-inhibiting activity that is equivalent to or better than that of rutin. The anthocyanin of the present invention is useful for treating complications related to diabetes and aging-related complications including renal disease, nerve injury, atherosclerosis, retinopathy, dermatologic symptom, and colored tooth resulting from AGE previously formed at a high concentration Furthermore, in addition to such usefulness, the anthocyanin of the present invention also has aldose-reductase-inhibiting activity. Hence, the peripheral effects of diabetes or galactosemia (e.g., macular oedama, cataract, retinopathy, neuropathy, and impaired neural conduction) can be prevented or treated using the anthocyanin of the present invention.

### Best Mode of Carrying out the Invention

The present invention relates to an AGE inhibitor and an aldose reductase inhibitor that comprise anthocyanin as an active ingredient.

Anthocyanin that can be used as the inhibitor of the present invention can be obtained through extraction from those containing anthocyanin in large amounts such as berries including cassis (black currant), elderberry, cowberry, gooseberry, cranberry, salmonberry, thimbleberry, strawberry, huckleberry, blackberry, blueberry, whortleberry, boysenberry, mulberry, raspberry, redcurrant, and loganberry, purple sweet potato, red cabbage, grape juice or pericarp, grape pericarp, purple corn, red radish, Perilla, red rice, dark sweet cherry, cherry, Hibiscus, plum, purple sweet potato, and purple yam. For example, compounds extracted from plant bodies and the like as the present inventors have disclosed in WO01/01798 can be used. Of these, anthocyanin to be used in the present invention is preferably extracted from berries, because raw materials for anthocyanin are expensive. Furthermore, for example, crystallized anthocyanin such as -3-glucoside and rutinoside of delphinidin and cyanidin as the present inventors have disclosed in (WO02/22847) are also preferable. Anthocyanin and crystallized anthocyanin that are used in the present invention can be obtained according to WO01/01798 or WO02/22847. The general formula of the anthocyanin of the present invention is as described above. The aglycon portion that can be used herein may be delphinidin, cyanidin, malvidin, pelargonidin, peonidin, or petunidin. Of these, delphinidin and cyanidin are preferable. Furthermore, the sugar portion that can be used herein may be glucose, rutinose, arabinose, galactose, or sophorose. Previous studies have shown that such compounds are atoxic and are present in blood. These compounds can be administered or ingested orally, parenterally, or rectally.

An AGE inhibitor comprising the anthocyanin of the present invention exhibits inhibition activity that is clearly better than that of aminoguanidine (aminoguanidine is known as a substance having AGE-formation-inhibiting activity) in an *in vitro* AGE formation inhibition test. Examples of such *in vitro* AGE formation inhibition test include a test using lysozyme and a test using bovine serum albumin (BSA) and fructose. Another example of such method is a method of Morimitu et al (Morimitu. Y. et. al., Biosci. Biotech. Biochem., 59, 2018-2021 (1995)). Activity can be compared using a concentration (IC₅₀) that inhibits AGE formation by 50% as an index. As described above, such AGE inhibitor comprising the anthocyanin of the present invention has excellent AGE formation-inhibiting activity and can be effectively used as a pharmaceutical preparation such as a preventive and therapeutic agent against diseases in which AGE is involved.

Furthermore, an aldose reductase inhibitor comprising the anthocyanin of the present invention exhibits inhibition activity equivalent to that of rutin (rutin is known as a substance having aldose-reductase-inhibiting activity) in an *in vitro* aldose-reductase-inhibiting activity test. An example of such *in vitro* aldose-reductase-inhibiting activity test is a method of Dufrane et al (Dufrane S.P. et al., Biochem. Med., 32, 99-105 (1984)). Activity can be compared using a concentration (IC₅₀) that inhibits aldose reductase activity by 50% as an index. As described above, such aldose reductase inhibitor comprising the anthocyanin of the present invention has excellent aldose-inhibiting activity and can be effectively used as a pharmaceutical preparation such as a preventive and therapeutic agent against diseases in which aldose reductase is involved.

Furthermore, anthocyanin used in the present invention has both AGE-formation-inhibiting activity and aldose-reductase-inhibiting activity. Thus, such anthocyanin of the present invention can be used as an inhibitor of AGE and aldose reductase.

Such an inhibitor of AGE and aldose reductase comprising the anthocyanin of the present invention is useful as a medicament and in particular an AGE formation inhibitor. Further particularly, such an inhibitor of AGE and aldose reductase is very useful as a preventive and therapeutic agent against: diabetic complications such as coronary heart disease, peripheral circulatory disturbance, cerebrovascular disorder, diabetic neuropathy, diabetic nephropathy, diabetic retinopathy, arterial sclerosis, arthrosclerosis, cataract, retinopathy, coagulopathy, and diabetic osteopenia; senile diseases such as atherosclerosis, glomerular nephritis, cataract, osteoarthrosis, periarticular rigidity, arthrosclerosis, senile osteoporosis, and Alzheimer's disease; and, since an active oxygen species is produced by the advanced Maillard reaction as is well known, diseases whose major cause is thought to be active oxygen, such as arteriosclerosis, coronary heart disease, cerebrovascular disorder, hepatic failure, renal failure, cataract, retinopathy, and autoimmune disease. Moreover, the Maillard reaction also proceeds in foods containing protein or amino acid, so that the protein or amino acid are deteriorated by the resulting AGE. Hence, also in foods, the inhibitor of AGE and aldose reductase is also useful as a compound that inhibits the Maillard reaction.

An AGE inhibitor comprising the anthocyanin of the present invention has inhibition activity almost equivalent to that of rutin that is a useful inhibitor, as discovered by the present inventors in an aldose-reductase-inhibiting activity test. As described above, such AGE inhibitor comprising the anthocyanin of the present invention also has aldose-reductase-inhibiting activity. Thus, with the use of such AGE inhibitor, peripheral effects of diabetes or galactosemia (macular oedama, cataract, retinopathy, neuropathy, and impaired neural conduction) caused by excessive accumulation of sorbitol or galactitol can also be prevented or treated.

Such an inhibitor of AGE and aldose reductase comprising the anthocyanin of the present invention forms salts with many inorganic acids and organic acids. This property is used to provide a form for production of a pure substance or to provide a form for administration of the inhibitor as a pharmaceutical preparation. Specifically, in the case of production, the inhibitor can be solubilized in a polar solvent such as water, extracted and purified, and isolated in the form of a salt showing preferable physicochemical properties through being adjusted to acidic In the case of application as a medicament, the inhibitor can be prepared in the form of a pharmacologically acceptable salt. Examples of possible forms of salts include acid addition salts formed of inorganic acid such as hydrochloric acid, nitric acid, hydrobromic acid, or sulfuric acid and salts induced with the use of atoxic organic acid such as aliphatic monocarboxylic acid, dicarboxylic acid, hydroxy alkanoic acid, hydroxyl alkanedioic acid, or amino acid or aromatic acid, aliphatic sulfonic acid, or aromatic sulfonic acid. Examples of such acid addition salts include hydrochloride, hydrobromate, nitrate, sulfate, hydrogensulfate, hydrogenphosphate, dihydrogen phosphate, acetate, propionate, tartrate, oxalate, malonate, succinate, fumarate, maleate, mandelate, benzoate, phthalate, methane sulfonate, benzene sulfonate, toluene sulfonate, citrate, lactate, malate, and glycolate. The above listed acid addition salts also have significance as pharmacologically acceptable pharmaceutical preparations. As pharmaceutical preparations, such acid addition salts may be advantageous in drug formulation. Furthermore, when administered to a human body, such acid addition salts may exert usefulness in terms of dispersibility and absorptive properties.

A medicament that contains such an inhibitor of AGE and aldose reductase comprising the anthocyanin of the present invention as an active ingredient can be administered to humans or non-human animals via either an oral route or a parenteral route of administration (e.g., intravenous injection, intramuscular injection, subcutaneous administration, rectal administration, and percutaneous administration). Hence, such pharmaceutical preparation that contains as an active ingredient the inhibitor of AGE and aldose reductase comprising the anthocyanin of the present invention can be formulated into an appropriate dosage form for each route of administration. Specifically, examples of an oral agent include tablets, capsules, powders, granules, and syrups. Examples of a perenteral agent include injections such as intravenous injections and intramuscular injections, agents for rectal administration, oleaginous suppositories, and aqueous suppositories. Each of these various pharmaceutical preparations can be produced by a standard method using a generally used vehicle, a disintegrating agent, a binder, a lubricant, a coloring agent, or the like.

Examples of the aforementioned vehicle include lactose, glucose, corn starch, sorbit, and crystalline cellulose. Examples of the aforementioned disintegrating agent include starch, alginate sodium, gelatine powder, calcium carbonate, calcium citrate, and dextrin. Examples of the aforementioned binder include dimethyl cellulose, polyvinyl alcohol, polyvinyl ether, methyl cellulose, ethyl cellulose, gum Arabic, gelatin, hydroxypropyl cellulose, and polyvinylpyrrolidone. Examples of the aforementioned lubricant include talc, magnesium stearate, polyethylene glycol, and hardened plant oil. Furthermore, the above injections can be produced by adding a buffer, a pH adjuster, a stabilizer, or the like, if necessary.

When a composition that contains the inhibitor of AGE and aldose reductase comprising the anthocyanin of the present invention is used as a pharmaceutical preparation, the anthocyanin is generally approximately 0.1 wt.% to 50 wt.% of the whole composition and preferably the anthocyanin is approximately 0.1 wt.% to 20 wt.% of the same. The content differs depending on the dosage form. The dose is appropriately determined depending on each case in view of a patient's age, body weight, sex, disease type, severity of symptoms, and the like. In general, 1 mg to 1000 mg per day and preferably 1 mg to 200 mg per day of the anthocyanin is administered to an adult. Such dose is administered once a day or at several separate times a day.

Furthermore, when a composition that contains the inhibitor of AGE and aldose reductase comprising the anthocyanin of the present invention is used as food, such composition can be blended with food so that the content of the compound of the present invention ranges from 0.01 wt.% to 10 wt.% of the whole food. Specifically, examples of possible forms of such composition for food include powders, granules, liquids, and pastes. Examples of food and drink include candies, chewing gums, juice, powdery drinks, chocolate, sweets in the form of tablets, jelly-like food, jam, hard capsules, and soft capsules.

### Examples

### [Example 1] Preparation of composition containing anthocyanin

A composition containing anthocyanin was prepared by the method according to WO01/01798 as follows.

3 kg of commercial concentrated cassis juice (2.8% purity of anthocyanin with reference to solid content) was diluted in water and then prepared at a concentration of Bx.10 (solid content concentration: 10%). The diluted juice was filtered using filter paper to remove foreign matter. Membrane separation was performed using an apparatus wherein a negatively charged reverse osmosis membrane (produced by NITTO DENKO CORPORATION, NTR-7410) had been set. Separation was performed until the concentrate could not circulate. Water was added again for dilution, and then separation was performed again. At the stage at which the concentrate no longer circulated, separation was completed. The concentrate was spray dried, so that a powdered composition containing anthocyanin at a high level was obtained. The purity of anthocyanin in this composition was 14.1% with reference to solid content.

### [Example 2] Preparation of crystalline anthocyanin

Furthermore, anthocyanin was prepared as described below in a crystalline form from the composition according to the method of WO02/22847.

40 g of the powder obtained according to the method of Example 1 (where anthocyanin ingredients were: D3G (delphinidin-3-O-glucoside) 12.5%; D3R (delphinidin-3-O-rutinoside) 47.9%; C3G (cyanidin-3-O-glucoside) 4.1%; and C3R (cyanidin-3-O-rutinoside) 35.5%, respectively) was fractionated using an ODS silica gel column and a 9% acetonitrile aqueous solution containing 0.1% TFA.

The thus obtained D3G fraction was 1.51 g, the C3G fraction was 0.98 g, the C3R fraction was 162 mg, and the D3R fraction was 231 mg.

Each concentrate was dissolved in a 5% hydrogen chloride/methanol solution and then allowed to stand at 5°C for 24 hours for crystallization. Thus, 1.06 g of crystalline D3G hydrochloride, 0.59 g of crystalline C3G hydrochloride, 58 mg of crystalline C3R hydrochloride, and 88 mg of crystalline D3R hydrochloride were prepared.

### [Test example 1] AGE formation suppression test

The test was conducted by a partially modified version of the method of Morimitu et al (Morimitu. Y. et. al., Biosci. Biotech. Biochem., 59, 2018-2021 (1995)). Specifically, glucose, each test substance dissolved in DMSO, and bovine serum albumin were incubated in a 67 mmol/L phosphate buffer (pH 7.2) at 60°C for 48 hours. AGE was thus sufficiently formed by incubation. The amount of formed AGE was discovered by dilution of the reaction solution followed by measurement using a fluorospectrophotometer. The 50% inhibition concentration (IC₅₀) was calculated using the thus obtained value. The lower the IC₅₀, the higher the AGE-inhibiting activity. This indicates the usefulness of a substance as an inhibitor.

The test substances used herein were the anthocyanin-containing composition prepared in Example 1 and 4 types of crystalline anthocyanin prepared in Example 2.

The results are as listed in the following table. It was revealed that the 4 types of crystalline anthocyanin used in this test have AGE-inhibiting activity stronger than that of aminoguanidine, which is known as a substance having strong AGE-inhibiting activity. Similarly, it was also revealed that the anthocyanin-containing compositions also have strong AGE-inhibiting activity.

**Table 1**

| Test substance | IC₅₀ |
|---|---|
| Example 1 | 200 µg/mL |
| Example 2 (D3R) | 163 µmol/L |
| Example 2 (D3G) | 99 µmol/L |
| Example 2 (C3R) | 154 µmol/L |
| Example 2 (C3G) | 132 µmol/L |
| Aminoguanidine | 1200 µmol/L |

### [Test example 2] Aldose-reductase-inhibiting activity test

The test was conducted by a partially modified version of the method of Dufrane et al (Dufrane S.P. et al., Biochem. Med., 32, 99-105 (1984)). Specifically, the lenses of Wistar male rats were homogenized with 10 mmol/L 2-mercapto ethanol. The resultant was centrifuged, and then the supernatant was used as a crude enzyme solution. Each test substance was dissolved in DMSO and then added to a 180 mmol/L phosphate buffer (pH 7.0). 100 mmol/L lithium sulfate and 0.03 mmol/L NADPH were further added to the solution to initiate a reaction at 30°C using 1 mmol/L DL glycerylaldehyde as a substrate. 0.5 mol/L HCl was added to stop the reaction. 6 mol/L sodium hydroxide containing 10 mmol/L imidazole was added to the solution. The solution was heated for 20 minutes at 60°C to convert NADP to a fluorescent substance. The thus generated fluorescent substance was subjected to measurement using a fluorospectrophotometer. 50% inhibition concentration (IC₅₀) was calculated using the thus obtained value. Similar to the result in Example 1, this result also indicated that the lower the IC₅₀, the stronger the inhibition activity.

The test substances used herein were the anthocyanin-containing composition prepared in Example 1 and 4 types of crystalline anthocyanin prepared in Example 2.

The results are as listed in the following table. It was revealed that the 4 types of crystalline anthocyanin used in this test have aldose-reductase-inhibiting activity that is equivalent to that of rutin, which is known as a substance having strong aldose-reductase-inhibiting activity. Similarly, it was also revealed that the anthocyanin-containing compositions also have strong aldose-reductase-inhibiting activity.

**Table 2**

| Test substance | IC₅₀ |
|---|---|
| Example 1 | 20 µg/mL |
| Example 2 (D3R) | 15.2 µmol/L |
| Example 2 (D3G) | 12.5 µmol/L |
| Example 2 (C3R) | 2.5 µmol/L |
| Example 2 (C3G) | 4.7 µmol/L |
| Rutin | 9.0 µmol/L |

All publications, patents, and patent applications cited herein are incorporated herein by reference in their entirety.

## Claims

1. An inhibitor of the formation of advanced glycation end products, which comprises anthocyanin.

2. The inhibitor of the formation of advanced glycation end products according to claim 1, wherein the anthocyanin is glycoside of delphinidin or cyanidin.

3. An inhibitor of aldose reductase, which comprises anthocyanin.

4. The inhibitor of aldose reductase according to claim 3, wherein the anthocyanin is glysocide of delphinidin or cyanidin.

5. A composition, which comprises anthocyanin and has activity of inhibiting advanced glycation end product formation and aldose-reductase-inhibiting activity.

6. A composition, which contains the inhibitor of the formation of advanced glycation end products according to claim 1 or 2 or the inhibitor of aldose reductase according to claim 3 or 4.

7. The composition according to claim 6, which is a medicament containing anthocyanin prepared from berries.

8. The composition according to claim 6, which is a medicament containing anthocyanin prepared from cassis.

9. The composition according to claim 6, which is a food containing anthocyanin prepared from berries.

10. The composition according to claim 6, which is a food containing anthocyanin prepared from cassis.

11. A preventive or therapeutic food against disease caused by the formation of advanced glycation end products or aldose reductase activity, which comprises anthocyanin.

12. The preventive or therapeutic food according to claim 11, wherein the above disease is diabetes or a related complication thereof.
